Europäisches Patentamt

(19) European Patent Office

Office européen des brevets

(11) Numéro de publication : **0 362 342 B1**

(12) **FASCICULE DE BREVET EUROPEEN**

(45) Date de publication du fascicule du brevet :
**27.11.91 Bulletin 91/48**

(51) Int. Cl.⁵ : **B25J 17/02, F16H 21/00**

(21) Numéro de dépôt : **89903759.2**

(22) Date de dépôt : **15.03.89**

(86) Numéro de dépôt international :
**PCT/FR89/00112**

(87) Numéro de publication internationale :
**WO 89/09120 05.10.89 Gazette 89/24**

---

(54) **DISPOSITIF ARTICULE, NOTAMMENT UTILISABLE DANS LE DOMAINE DE LA ROBOTIQUE.**

---

(30) Priorité : **21.03.88 FR 8803630**

(43) Date de publication de la demande :
**11.04.90 Bulletin 90/15**

(45) Mention de la délivrance du brevet :
**27.11.91 Bulletin 91/48**

(84) Etats contractants désignés :
**BE DE GB IT LU NL**

(56) Documents cités :
**FR-A- 2 549 916
INTERNATIONAL JOURNAL OF ROBOTICS
RESEARCH, vol. 5, no. 2, 1986, Cambridge
Massachusetts US, pages 157 - 182; E.F. FICH-
TER: "A Stewart platform-based manipulator:
General theory and practical construction"**

(73) Titulaire : **INRIA INSTITUT NATIONAL DE
RECHERCHE EN INFORMATIQUE ET EN
AUTOMATIQUE
Domaine de Voluceau, B.P. 105
Rocquencourt F-78153 Le Chesnay (FR)**

(72) Inventeur : **MERLET, Jean-Pierre
Bâtiment B2 Résidence Crista-Galli
Route de Saint-Jean F-06600 Antibes (FR)**

(74) Mandataire : **Plaçais, Jean-Yves et al
Cabinet Netter, 40, rue Vignon
F-75009 Paris (FR)**

---

## Description

L'invention concerne un dispositif articulé du type comprenant deux plateaux reliés entre eux par des segments articulés conformés selon une structure en treillis fermé et un ensemble de commande de la position relative de ces deux plateaux.

Dans l'art antérieur, un tel dispositif, appelé manipulateur parallèle possède généralement six segments de longueur variable articulés sur les plateaux par des joints universels et un ensemble de commande qui permet d'obtenir jusqu'à six degrés de liberté d'un plateau par rapport à l'autre. Il est utilisable notamment dans le domaine de la robotique où il peut constituer par exemple l'extrémité d'un bras de robot d'insertion ou d'assemblage ou encore il peut constituer une structure solidaire d'une plateforme fixe pour faire face au bras d'un robot. Dans cette application le dispositif constitue alors une "main gauche" pour le robot qui lui fait face.Dans tous les cas, le dispositif doit permettre la réalisation de tâches complexes d'assemblage fin, d'usinage de précision ou le contrôle de surfaces par contact.

Un dispositif de ce type peut trouver également une application dans le domaine des simulateurs dynamiques de mouvement, tels que les simulateurs de vol ou de conduite d'engins.

On connaît par FR-A-2549 916, un dispositif de ce type qui comporte deux plateaux reliés entre eux par six vérins pneumatiques constituant les vérins articulés à la fois sur le plateau supérieur et inférieur, et commandés par des électrovannes. Ce type de manipulateur, doué d'une élasticité naturelle donnée par les vérins pneumatiques, présente un grand intérêt pour la robotique en raison de sa grande précision de positionnement et de l'intégration aisée des mesures de forces. Mais un tel dispositif présente un encombrement relativement important ainsi qu'une masse et une inertie de l'équipage mobile ce qui limitent sa vitesse de déplacement. Cet encombrement important est lié à la présence des vérins pour la motorisation des segments articulés et ceux-ci limitent en outre l'espace de travail du dispositif en raison de leur diamètre. Enfin, les vérins pneumatiques nécessitent une dépense d'énergie même sans mouvaient du plateau supérieur.

On connaît égalaient par le document de Jean-Pierre MERLET "Parallel Manipulator, Part 1 : Theory, Design, Kinematics and Control", INRIA research Report, n° 646, Mars 1987, ci-après dénommé document MERLET, un dispositif plus particulièrement destiné à assurer les fonctions de "main gauche" pour un robot. Ce dispositif est constitué également de deux plateaux reliés par des segments articulés à la fois sur le plateau supérieur et sur le plateau inférieur.Chaque segment de ce dispositif est muni d'un organe élastique qui permet une faible modification de longueur des segments sous l'influence des forces exercées sur le plateau supérieur. La géométrie du dispositif peut ainsi être légèrement modifiée sous l'influence des forces appliquées et le système est dit à "compliance passive". La mobilité du plateau supérieur est assurée par la possibilité de commander dans une certaine mesure la longueur de chacun des segments. Cette fonction est obtenue par la présence d'un vérin électrique dans chacun des segments. Cependant, ces vérins électriques ont l'inconvénient de présenter une importante longueur pour de faibles variations possibles de celle-ci. Ainsi, typiquement, pour une variation de longueur des segments d'au plus 5 cm, la longueur des segments est de 50 cm ce qui conduit à une hauteur globale du système d'environ 50 cm. Cette caractéristique impose alors un encombrement assez important. De plus la masse des vérins électriques actuellement commercialisés conduit à une masse totale du dispositif assez élevée, de l'ordre de 11 kg. Ceci est incompatible avec les capacités de charge des robots les plus couramment répandus et on ne peut donc pas utiliser ce dispositif comme extrémité de bras d'un robot d'insertion ou d'assemblage par exemple.

La présente invention remédie à ces inconvénients en proposant un dispositif qui permet d'obtenir, outre une haute précision de positionnement et une forte charge nominale, un encombrement total réduit et une masse et une inertie réduites de l'équipage mobile.

Un autre but de l'invention est d'obtenir un encombrement minimal des segments ce qui permet d'obtenir un espace de travail accru pour les degrés de liberté en rotation.

L'invention a encore pour but de permettre un libre choix de la longueur des segments.

Un autre but de l'invention est d'obtenir une compliance passive du dispositif c'est-à-dire qui permette de calculer la position et l'orientation exactes du plateau mobile en dépit des déplacements dus à la compliance passive du dispositif.

L'invention a encore pour but d'effectuer une mesure permanente des forces exercées sur le plateau mobile.

La présente invention a donc pour objet un dispositif articulé comprenant un plateau inférieur, un plateau supérieur et un ensemble de commande de la position relative des deux plateaux ledit ensemble de commande comportant :

- trois paires d'emplacements d'articulation rapprochés situées sur le plateau supérieur,
- trois paires d'emplacements de liaison rapprochés situées sur le plateau inférieur,
- six organes de liaison possédant chacun une extrémité supérieure articulée sur l'un respectif des emplacements d'articulation, et une extrémité inférieure reliée par une articulation inférieure à l'un respectifs des emplacements de liaison, de telle sorte que deux emplacements d'articulation d'une même paire sont reliés, par deux organes de liaison, aux deux emplacements de liaison consécutifs, qui sont respectivement les plus proches desdits deux emplacements d'articulation, et qui appartiennent à deux paires d'emplacements de liaison différentes,

– des moyens moteurs pour faire varier indépendamment sur commande la position d'un emplacement d'articulation par rapport au plateau inférieur,

caractérisé en ce que chaque organe de liaison a une longueur sensiblement constante et en ce que l'ensemble de commande comprend en outre, six organes de coulissement respectivement reliés, d'une part, aux six emplacements de liaison et, d'autre part, par l'intermédiaire des six articulations inférieures aux extrémités inférieures des six organes de liaison, chaque organe de coulissement étant propre à coulisser relativement au plateau inférieur selon une direction de coulissement prédéterminée, sous l'action des moyens moteurs.

Dans un mode de réalisation, toutes les directions de coulissement sont sensiblement parallèles et sensiblement orthogonales au plan contenant les trois paires d'emplacements de liaison situées sur le plateau inférieur.

Avantageusement, chaque organe de liaison est susceptible, sous l'action des moyens moteurs, d'être positionné dans un cône de révolution ayant pour axe ladite direction de coulissement et pour sommet ladite articulation inférieure.

L'ensemble de commande peut comprendre également des moyens de blocage pour bloquer chaque organe de coulissement en rotation autour de la direction de coulissement.

Ces moyens de blocage peuvent comprendre des moyens de blocage supérieurs pour bloquer au niveau de l'emplacement d'articulation correspondant, chaque organe de liaison en rotation autour d'un axe reliant ses extrémités inférieure et supérieure; dans ce cas, chaque articulation inférieure est de préférence un joint de cardan constituant des moyens de blocage inférieurs.

Dans un mode de réalisation, chaque organe de coulissement possède des moyens filetés et les moyens moteurs comprennent six vis sans fin associées respectivement aux six organes de coulissement et respectivement mues en rotation autour de leur axe longitudinal par six moteurs; dans ce cas, les moyens filetés d'un organe de coulissement et la vis sans fin associée coopèrent avantageusement pour permettre le coulissement de l'organe de coulissement selon ladite direction de coulissement.

Chaque organe de coulissement comprend, de préférence, un tube cylindrique creux, possédant une première extrémité reliée à l'articulation inférieure correspondante, ouvert à son autre extrémité, et les moyens filetés comprennent avantageusement un écrou fileté, solidairement lié en translation et en rotation à ladite autre extrémité et coopérant avec la vis sans fin.

Chaque tube cylindrique creux coulisse dans une douille à billes reliée au plateau inférieur.

Dans un mode de réalisation, chaque emplacement d'articulation est propre à définir une première articulation supérieure possédant un premier axe de rotation sensiblement perpendiculaire au plan contenant les trois paires d'emplacements d'articulation, une deuxième articulation supérieure possédant un deuxième axe de rotation sensiblement orthogonal audit premier axe de rotation et concourant avec ledit premier axe de rotation et un troisième axe de rotation sensiblement orthogonal au deux premiers axes et concourant avec ceux-ci, ce qui permet d'obtenir trois degrés de liberté en rotation.

Les trois points milieu des trois paires d'emplacements d'articulation sont situés respectivement aux trois sommets d'un premier triangle équilatéral et les six emplacements de liaison sont situés sur un cercle.

Dans la position de repos, les trois points situés respectivement au milieu des trois arcs de cercle reliant les deux emplacements de liaison respectifs des trois paires, sont placés respectivement aux trois sommets d'un second triangle équilatéral, décalé par rapport au premier triangle équilatéral d'un angle de 60° autour de l'axe reliant les isobary-centres respectifs des deux triangles équilatéraux.

Dans un mode de réalisation préféré les deux emplacements d'articulation de chaque paire du plateau supérieur sont confondus.

L'ensemble de commande peut comprendre également des moyens de détection d'au moins une grandeur mécanique représentative de l'état du dispositif.

Ces moyens de détection comprennent avantageusement six premiers capteurs, permettant de déterminer la variation de la position respective de chaque emplacement d'articulation par rapport au plateau inférieur.

Les moyens de détection peuvent comprendre également six capteurs d'effort permettant de déterminer l'effort respectif s'exerçant sur chaque organe de liaison.

Avantageusement l'ensemble de commande comprend des moyens de traitement recevant les informations des moyens de détection et commandant les moyens moteurs. Afin d'assurer la compliance du dispositif, l'ensemble de commande peut comprendre six organes élastiques amortisseurs reliés respectivement aux six organes de liaison.

Dans un mode de réalisation préféré, chaque premier capteur est un capteur de déplacement, notamment un potentiomètre linéaire, relié d'une part à l'articulation inférieure et d'autre part au support inférieur, l'organe élastique correspondant étant situé sur l'organe de coulissement correspondant.

L'ensemble de commande comprend de préférence des moyens de sécurité limitant le déplacement de chaque articulation inférieure par rapport au plateau inférieur.

Avantageusement chaque moyen de sécurité comprend des organes de coupure d'alimentation des moyens moteurs assurant également après coupure d'alimentation, le freinage de l'organe de coulissement correspondant.

D'autres avantages et caractéristiques de l'invention apparaîtront à l'examen de la description détaillée ci-après et des dessins annexés sur lesquels :

 – la figure 1 est une représentation très schématique d'un mode de réalisation général du dispositif selon l'invention,

 – la figure 2 est une représentation schématique en perspective d'un mode de réalisation préféré du dispositif selon l'invention,

 – la figure 3 est une vue de dessus schématique des principaux constituants du dispositif de la figure 2

 – la figure 4 est une vue en perspective partielle d'un emplacement de liaison du dispositif de la figure 2,

 – la figure 5 est la coupe V-V de la figure 4,

 – la figure 6 est une représentation schématique d'un moyen de sécurité du dispositif de la figure 2,

 – la figure 7 est une vue en élévation avec parties arrachées d'un emplacement d'articulation du dispositif de la figure 2, et,

 – la figure 8 est un synoptique schématique des moyens de traitement du dispositif de la figure 2.

Les dessins comportant pour l'essentiel des éléments de caractère certain font partie intégrante de la description et pourront servir non seulement à mieux faire comprendre la description détaillée ci-après mais aussi contribuer le cas échéant à la définition de l'invention.

On décrira ci-après un dispositif plus particulièrement destiné à constituer une extrémité de bras de robot, bien que l'invention ne soit pas limitée au domaine de la robotique, ainsi qu'il a été expliqué ci-avant.

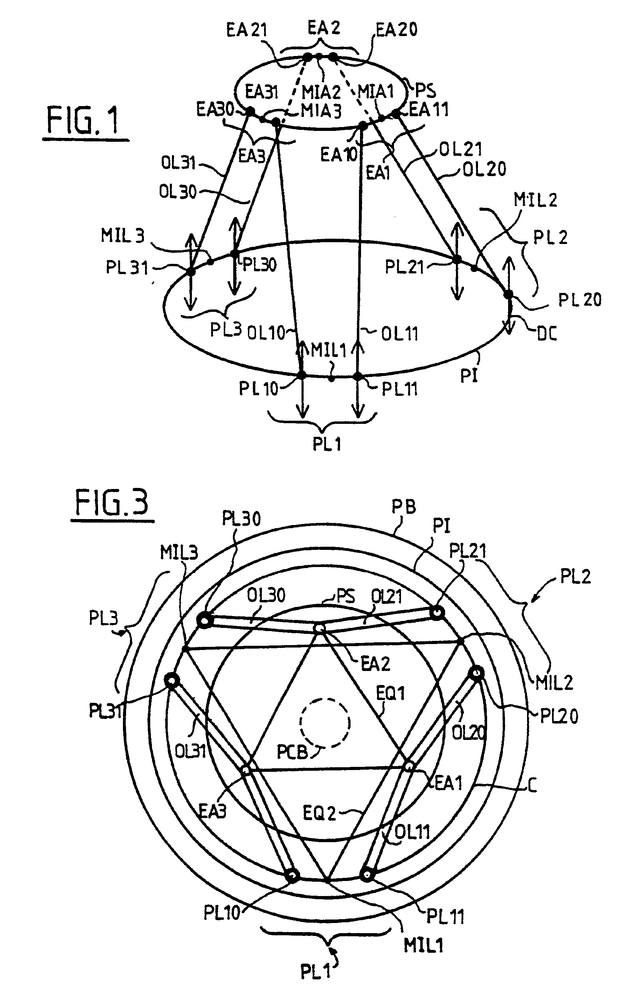

La figure 1 montre de façon générale la structure d'un dispositif selon l'invention. Ce dispositif comprend un plateau supérieur PS et un support inférieur PI reliés par six organes de liaison OL10, OL11, OL20, OL21, OL30, OL31, articulés d'une part sur le plateau supérieur et d'autre part sur le plateau inférieur. Le plateau inférieur PI comprend trois paires d'emplacements de liaison rapprochées PL1, PL2, PL3, et le plateau supérieur PS comprend trois paires d'emplacements d'articulation rapprochés EA1, EA2, EA3. Les six organes de liaison possèdent chacun une extrémité supérieure articulée sur un emplacement d'articulation et une extrémité inférieure reliée, par une articulation inférieure non représentée sur cette figure, à un emplacement de liaison.

D'une façon générale, dans un but de faciliter la compréhension des références, l'indice $i$ se rapportera à une paire d'emplacements de liaison ou d'emplacements d'articulation et l'indice $j$ se rapportera à l'un des éléments d'une paire $i$. Lorsqu'il sera nécessaire de différencier les deux éléments d'une même paire, ceux-ci seront affectés respectivement de l'indice $j_1$ et de l'indice $j_2$, et lorsqu'il sera nécessaire de différencier deux paires, celles-ci seront affectées respectivement des indices $i_1$ et $i_2$.

La structure du dispositif selon l'invention est telle que deux emplacements d'articulation $EAij_1$ et $EAij_2$ appartenant à une paire $EAi$ d'emplacements d'articulation rapprochés, sont reliés respectivement, par deux organes de liaison $OLi_{1j2}$, $OLi_{2j1}$, à deux emplacements de liaison consécutifs $PLi_{1j2}$ et $PLi_{2j1}$, qui sont respectivement les plus proches desdits deux emplacement d'articulation $EAi_{j1}$ et $EAi_{j2}$, ces deux emplacements de liaison appartenant respectivement à deux paires $PLi_1$ et $PLi_2$ d'emplacements de liaison différentes.

Chaque articulation inférieure correspondant à chaque organe de liaison peut coulisser par rapport au plateau inférieur selon une direction de coulissement DC passant par l'emplacement de liaison correspondant. Dans un but de simplicité de réalisation, toutes les directions de coulissement DC sont sensiblement parallèles entre elles et sensiblement orthogonales au plan contenant les trois paires d'emplacements de liaison PLi. Ce coulissement des articulations inférieures est obtenu à l'aide de moyens moteurs non représentés sur cette figure.

On définit maintenant une position de repos du dispositif qu'a celui-ci lorsque les deux plateaux PI et PS sont parallèles et qu'aucune action n'est ou n'a été effectuée sur les organes de liaison par l'intermédiaire des moyens moteurs. Ainsi dans cette position de repos la structure de liaison entre les deux plateaux est telle que deux organes de liaison issus de deux emplacements d'articulation différents d'une même paire, divergent vers deux emplacements de liaison appartenant à deux paires d'emplacements de liaison différentes $PLi_1$ et $PLi_2$. Ainsi, par exemple, la droite joignant les deux emplacements d'articulation EA10 et EA11, les deux organes de liaison OL11 et OL20 ainsi que la droite joignant les deux emplacements de liaison PL11 et PL20 forment un trapèze dont la grande base se situe au niveau du plateau inférieur et dont la petite base, se situe au niveau du plateau supérieur.

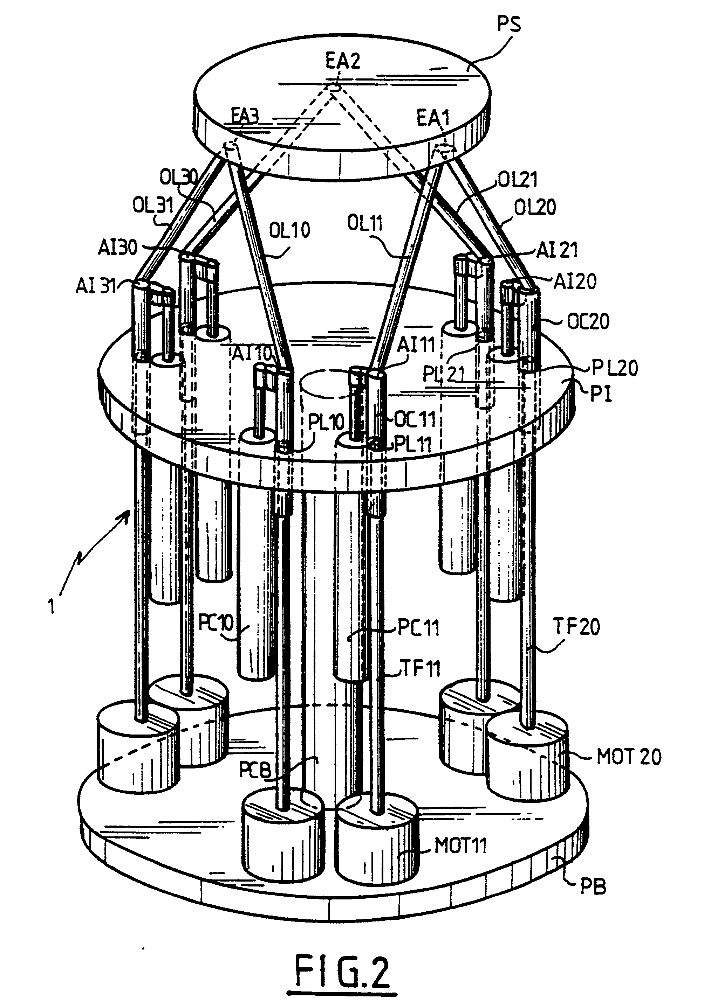

Il est maintenant fait référence plus particulièrement aux figures 2 à 9 sur lesquelles est représenté un mode de réalisation préféré du dispositif de la figure 1.

Les plateaux inférieur et supérieur du dispositif 1 de la figure 2 sont circulaires, le plateau supérieur ayant un diamètre inférieur à celui du plateau inférieur. Le dispositif 1 comprend également un plateau de base PB d'un diamètre légèrement supérieur à celui du plateau inférieur PI. Le plateau PI est situé entre le plateau PS et le plateau PB et ce dernier est solidaire du plateau inférieur PI par l'intermédiaire d'un pied central PCB. Les centres respectifs des trois plateaux sont situés sur une même droite.

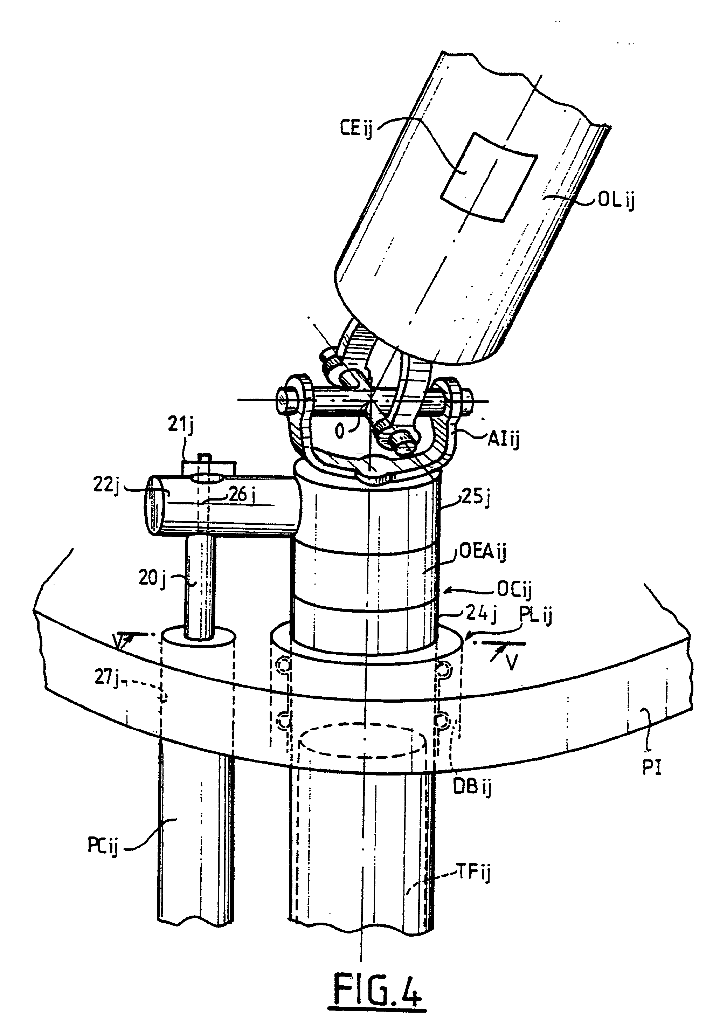

Si l'on se réfère à la figure 3, on voit que les six emplacements de liaison PLij sont situés sur un cercle C ayant pour centre celui du plateau PI. Les deux emplacements d'articulation d'une même paire EAi sont en fait confondus. Cette disposition permet un gain de masse et simplifie les calculs pour le contrôle du dispositif qui sera détaillé ci-après. Ainsi, dans la suite du texte un emplacement d'articulation sera désigné par la référence EAi. Sur cette figure donc, les points milieu MIAi de deux emplacements d'articulation EAij d'une paire d'emplacements d'articulation EAi sont confondus et notés également EAi. Ces trois emplacements d'articulation EAi sont disposés respectivement sensiblement au sommet d'un triangle équilatéral EQ1 dont l'isobarycentre est situé au centre du plateau supérieur. De même, les points milieu MILi des arcs de cercle reliant deux emplacements de liaison PLij sont situés sensiblement respectivement au sommet d'un second triangle équilatéral EQ2 ayant pour isobarycentre le centre du plateau inférieur PI et décalé par rapport au premier triangle équilatéral EQ1 d'un angle d'environ 60° autour de l'axe passant par les isobarycentres respectifs des deux triangles équilatéraux.

Bien entendu, le caractère équilatéral des triangles EQ1 et EQ2 décrit dans cet exemple n'est pas limitatif.

Si l'on se réfère plus particulièrement à la figure 2 on voit qu'un emplacement de liaison PLij comprend un alésage dans lequel est susceptible de coulisser un organe de coulissement OCij dont une extrémité est reliée par l'intermédiaire de l'articulation inférieure Alij à l'extrémité inférieure de l'organe de liaison OLij. Cette extrémité de l'organe de coulissement est toujours située entre le plateau inférieur et le plateau supérieur. L'extrémité supérieure de l'organe de liaison OLij est articulée sur l'emplacement d'articulation EAi. Cet emplacement d'articulation EAi permet également l'articulation d'un organe de liaison reliant l'emplacement de liaison voisin de l'emplacement de liaison PLij et appartenant à une paire d'emplacements de liaison différente.

L'organe de coulissement OCij est également lié, par l'intermédiaire de son extrémité proche de l'articulation inférieure Alij à un premier capteur PCij dont la nature et la fonction seront explicitées ci-après. Ce capteur PCij est solidairement fixé sur le plateau inférieur dans une zone proche de l'emplacement de liaison PLij.

L'extrémité libre d'une vis sans fin TFij pénètre dans l'organe de coulissement OCij par son extrémité opposée à celle reliée à l'articulation inférieure Alij.

Cette vis sans fin TFij est reliée à son autre extrémité à un moteur d'entraînement MOTij comme par exemple un moteur couple fixé sur le plateau de base PB. Les fonctions de cette vis sans fin et de ce moteur seront explicitées ci-après. Tous les éléments constitutifs du dispositif 1, à l'exception des plateaux inférieur et supérieur, appartiennent à un ensemble de commande de la position relative des deux plateaux PI et PS, et ne sont pas tous représentés sur la figure 2 afin de ne pas l'encombrer.

Bien entendu les six sous-ensembles comprenant respectivement un moteur, une vis sans fin, un organe de coulissement, un premier capteur, une articulation inférieure et un organe de liaison sont identiques.

Si l'on se réfère maintenant à la figure 4 on voit que l'organe de coulissement OCij comprend un tube cylindrique creux 24j surmonté à une de ses extrémités par un organe élastique amortisseur OEAij lui-même surmonté d'une extension cylindrique 25j dont l'extrémité opposée à celle en contact avec l'organe OEAij supporte l'articulation inférieure Alij.

L'organe de liaison OLij comprend un capteur de force CEij destiné à mesurer la grandeur des forces mises en jeu par cet organe de liaison. Ce capteur de force peut être par exemple une jauge extensométrique.

L'extension cylindrique 25j comprend un bras 22j percé d'un trou cylindrique 26j d'axe parallèle à l'axe de l'organe de coulissement OCij et donc parallèle à la direction de coulissement DC. Dans ce trou cylindrique 26j vient s'enfiler la tige 20j du premier capteur PCij qui est en fait un potentiomètre linéaire. Cette tige 20j est solidaire du bras 22j par l'intermédiaire d'un écrou 21j. Le capteur PCij est solidairement fixé dans un autre trou cylindrique 27j du plateau inférieur, proche de l'emplacement de liaison PLij.

L'articulation inférieure Alij est un joint de cardan dont un axe est constamment sensiblement parallèle au plateau inférieur PI, l'autre axe étant bien entendu concourant avec le premier en un point O. Les axes longitudinaux respectifs des organes de coulissement OCij et de liaison OLij sont également concourants au point O.

Afin de limiter le frottement de coulissement, le tube creux 24j coulisse à travers le plateau inférieur PI dans une douille à billes DBij.

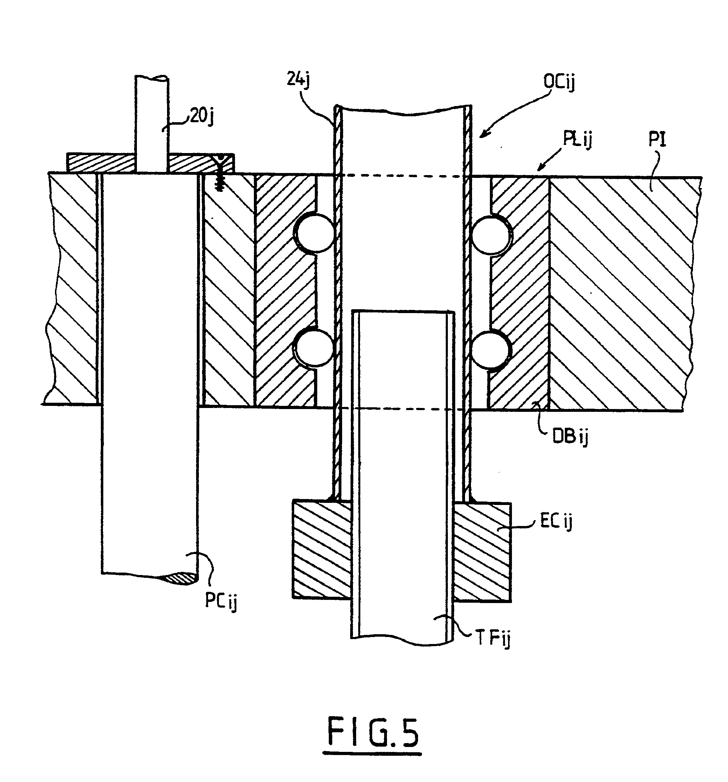

La liaison entre la vis sans fin TFij et le tube creux 24j de l'organe de coulissement OCij est représentée de façon détaillée sur la figure 5. Sur cette figure, on voit que le tube creux 24j est ouvert à son extrémité opposée à celle qui est en contact avec l'organe élastique amortisseur OEAij et comporte au niveau de cette ouverture un écrou fileté ECij qui lui est solidairement lié en translation et en rotation. Le filetage de la vis sans fin TFij coopère avec celui de l'écrou ECij lequel est muni d'un organe de rattrapage de jeu non représenté. La longueur de la vis sans fin TFij ainsi que la longueur du tube creux 24j ont été déterminées pour qu'en fonctionnement, la vis sans fin TFij ne vienne pas en butée contre l'extrémité du tube creux 24j qui est en contact avec l'organe élastique amortisseur OEAij.

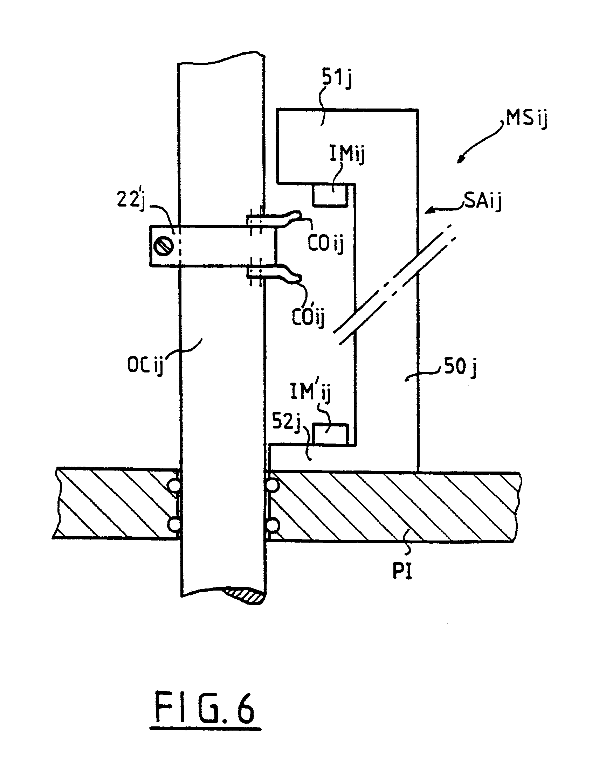

La figure 6 montre de façon schématique un moyen de sécurité MSij associé à un organe de coulissement OCij. Ce moyen de sécurité MSij comprend un support auxiliaire SAij ayant une partie 50j sensiblement orthogonale au plateau inférieur PI et prolongée par une extension 51j repliée d'équerre vers l'organe de coulissement OCij de façon à être sensiblement parallèle au plateau inférieur PI. Le support SAij comprend également une extension supplémentaire 52j située sur le plateau inférieur PI et repliée d'équerre par rapport à la partie 50j de façon à se situer en regard de l'extension 51j. La face de l'extension 51j située en regard du plateau inférieur PI comprend un premier interrupteur magnétique IMij tandis que la face de l'extension supplémentaire 52j située en regard du premier interrupteur IMij comprend un second interrupteur magnétique IM'ij. L'extrémité de l'organe de coulissement OCij située proche de l'articulation inférieure AIij, comporte un bras supplémentaire 22'j, opposé au bras 22j, muni de deux lames élastiques COij et CO'ij situées l'une au-dessus de l'autre et espacées l'une de l'autre. Ces deux lames COij et CO'ij sont agencées de façon à venir respectivement en contact avec les extensions 51j et 52j du support SAij. Le fonctionnement détaillé de ce mécanisme de sécurité sera décrit ci-après.

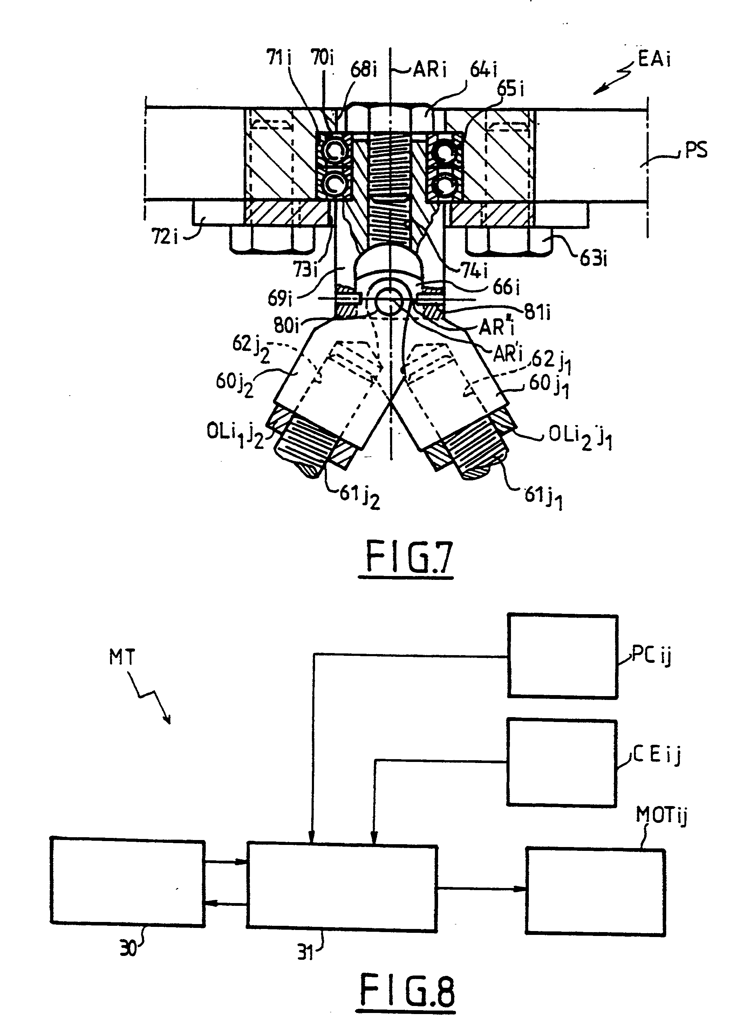

La figure 7 décrit de façon détaillée un emplacement d'articulation EAi situé sur le plateau supérieur PS. L'emplacement d'articulation EAi comprend un trou cylindrique 68i sensiblement perpendiculaire au plateau PS et débouchant sur la face supérieure de ce plateau PS définie comme étant celle opposée à celle située en regard du plateau inférieur PI. Ce trou cylindrique 68i a un axe longitudinal ARi, se prolonge par un épaulement annulaire 70i replié d'équerre en s'éloignant de l'axe ARi et se termine par une jupe cylindrique 71i coaxiale avec le trou cylindrique 68i. Cette jupe cylindrique 71i débouche sur la face inférieure du plateau supérieur PS.

La face inférieure du plateau PS comporte une plaque annulaire 72i percée en son centre d'un alésage 73i ayant également pour axe l'axe ARi, et un diamètre sensiblement égal au diamètre du trou cylindrique 68i. L'épaulement 70i, la jupe 71i ainsi que la plaque annulaire 72i servent de support pour les faces externes de deux roulements à billes 65i.

L'emplacement d'articulation EAi comprend également un corps 69i dont la partie supérieure vient s'appuyer sur les faces internes des deux roulements à billes 65i et dont la partie inférieure c'est-à-dire la partie la plus proche du plateau inférieur PI, comporte un U dont les deux branches sont parallèles à l'axe ARi. Ce corps 69i possède en son centre, dans sa partie supérieure, un alésage fileté 74i coopérant avec un boulon 64i le rendant solidaire du plateau PS par l'intermédiaire des roulements à billes 65i. La plaque annulaire 72i est rendue solidaire également du plateau PS par deux-boulons 63i. Le corps 69i est ainsi bloqué en translation selon l'axe ARi mais est libre en rotation autour de cet axe ARi.

Les deux extrémités libres des deux branches du U du corps 69i sont reliées par une chape 66i percée d'un trou dont l'axe AR'i est sensiblement orthogonal à l'axe ARi et concourant avec celui-ci. Ce trou reçoit un arbre 80i sur lequel viennent s'articuler deux douilles cylindriques $60j_1$ et $60j_2$ comportant respectivement en leur centre des alésages longitudinaux filetés $62j_1$ et $62j_2$ destinés à recevoir respectivement les extrémités filetées $61j_1$ et $61j_2$ des organes de liaison $OLi_2j_1$ et $OLi_1j_2$. La chape 66i est articulée sur les branches du corps 69i selon un axe AR"i sensiblement orthogonal aux axes ARi et AR'i et concourant avec ceux-ci.

Le corps 69i est donc propre à définir une première articulation supérieure possédant un premier axe de rotation ARi, l'arbre 80i est propre à définir une deuxième articulation supérieure possédant un second axe de rotation AR'i et l'arbre 81i est propre à définir une troisième articulation supérieure possédant un troisième axe de rotation ce qui permet d'obtenir trois degrés de liberté en rotation. On obtient alors une articulation globale de type rotule. La fixation des deux douilles cylindriques $60j_1$ et $60j_2$ sur l'emplacement d'articulation EAi ainsi que l'agencement des trois axes de rotation ARi, AR'i, AR"i interdisent toute possibilité de rotation propre des douilles cylindriques $60j_1$ et $60j_2$ autour de leur axe longitudinal, et la coopération des filetages $61j_1$ et $61j_2$ avec respectivement les alésages filetés $62j_1$ et $62j_2$ contribue à bloquer les organes de liaison $OLi_2j_1$ et $OLi_1j_2$ en rotation autour d'un axe liant leurs extrémités inférieure et supérieure. Cet agencement particulier de l'emplacement d'articulation ainsi que des différents filetages constituent des moyens de blocage supérieurs.

Puisque l'extrémité inférieure de chaque organe de liaison OLij est reliée à l'organe de coulissement OCij correspondant par un joint de cardan, et que chaque organe de liaison OLij est bloqué en rotation autour d'un axe reliant ses deux extrémités, le joint de cardan AIij interdit la rotation propre de l'organe de coulissement OCij autour de la direction de coulissement et donc constitue des moyens de blocage inférieurs.

Ainsi, la combinaison des moyens de blocage inférieurs et supérieurs constitue-t-elle des moyens de blocage de la rotation de l'organe de coulissement autour de la direction de coulissement. Bien entendu, on pourrait envisager d'autres types de moyens de blocage.

L'homme de l'art comprendra que l'agencement du joint de cardan Alij, de l'organe de coulissement correspondant OCij et de l'emplacement d'articulation correspondant EAi permet à l'organe de liaison OLij d'être positionné dans un cône de révolution ayant pour axe ladite direction de coulissement DC et pour sommet le joint de cardan Alij.

La figure 8 représente un synoptique schématique des moyens de traitement du dispositif décrit ci-dessus. Ces moyens de traitement comprennent un contrôleur 31 qui reçoit d'une part des informations issues des premiers capteurs PCij et des capteurs de force CEij, et qui dialogue avec un ordinateur central 30 pour commander les moteurs MOTij.

Le dispositif ainsi décrit a une masse d'environ 2 kg, chacun des organes de liaison pesant environ 100 g. Leur longueur constante est environ de 10 cm et les dimensions du dispositif sont telles que celui-ci est inclus dans un cylindre de révolution de rayon sensiblement égal à 8 cm et de hauteur sensiblement égale à 20 cm. L'homme de l'art comprendra donc que l'on a réalisé ainsi un dispositif d'un encombrement réduit et d'une masse faible parfaitement adapté à la micromécanique. La précision de positionnement absolue du dispositif est de l'ordre de 10 microns et sa charge nominale est. d'environ 25 kg. Le fait d'avoir utilisé des organes de liaison dépourvus de motorisation lui confère un espace de travail délimité, par un déplacement de 4 cm en vertical et de plus ou moins 4 cm en latéral, par une possibilité de rotation de plus ou moins 61° autour de l'axe joignant les centres respectifs des deux plateaux PI et PS et de plus ou moins 33° autour des axes orthogonaux à ce dernier. Cet espace de travail est nettement plus important que celui notamment décrit dans le document MERLET.

De plus la masse principale du dispositif est constituée par les moteurs MOTij. Cette conception est telle que cette masse se trouve en dessous du plateau inférieur. Le centre de gravité du dispositif est donc placé en dessous du plateau inférieur et non plus comme dans les dispositifs antérieurs, au milieu des segments articulés. Ceci est très favorable pour l'emploi de ce dispositif comme extrémité de bras de robot, car la charge utile du robot porteur n'est pas pénalisée. Enfin, l'absence de moyens moteurs au niveau des organes de liaison, permet de les changer sans difficultés, pour les adapter à tous types d'applications.

Le fonctionnement de ce dispositif va maintenant être expliqué en détail. Comme il a été expliqué ci-avant le but de ce dispositif est d'aider à la réalisation d'assemblages et notamment lorsque les jeux sont incompatibles avec la précision des manipulateurs classiques. Le robot porteur amène le dispositif dans la zone où se situent l'assemblage et le dispositif, qui tient une des pièces à assembler, corrige les erreurs de position en utilisant l'information fournie par les capteurs de force CEij. Le dispositif, ayant 6 degrés de liberté, permet la correction de tous les types d'erreurs de positionnement.

Pour effectuer ces déplacements, les organes de coulissement déplacent selon la direction de coulissement verticale DC les articulations inférieures Alij des organes de liaison selon les indications fournies par le contrôleur 31. Pour ce faire, chaque moteur MOTij entraîne en rotation la vis sans fin correspondante TFij. L'écrou ECij monté sur cette vis sans fin, solidaire de l'organe de coulissement OCij, entraîne celui-ci en translation. Ceci est possible car l'organe de coulissement OCij est bloqué en rotation autour de la direction de coulissement DC par les moyens de blocage. Bien entendu les moteurs MOTij sont commandés indépendamment.

Le déplacement linéaire de l'articulation inférieure Alij est mesuré par le potentiomètre linéaire PCij grâce à sa liaison par l'intermédiaire de sa tige 20j avec le bras 22j.

Cet entraînement direct de l'organe de coulissement OCij permet de réduire au minimum les jeux de transmission. La commande du moteur MOTij est effectuée à partir des indications du potentiomètre PCij et le contrôle en couple du moteur permet d'appliquer une force de consigne sur chaque organe de liaison OLij.

Réciproquement, si le plateau supérieur est soumis à une force, celle-ci se distribue sur chaque organe de liaison OLij selon la direction de cet organe. Chaque organe de coulissement OCij est alors soumis à une force radiale, qui est annulée par la douille à billes DBij, et à une force axiale. Cette force axiale est transmise à l'organe élastique amortisseur OEAij qui se comprime ou se dilate d'une quantité proportionnelle à cette force. Le plateau supérieur PS se déplace alors légèrement ce qui assure la compliance passive du dispositif. Le déplacement du plateau supérieur est mesuré par les potentiomètres PCij ce qui permet à l'ordinateur 30 de calculer la position exacte du mobile. L'homme de l'art comprendra alors que la position de l'organe élastique amortisseur OEAij sur l'organe de coulissaient est particulièrement avantageuse, comparée à un élément élastique qui serait inséré dans chaque organe de liaison car les déplacements dus à la compliance passive peuvent être mesurés de façon précise par les capteurs de déplacement. D'autre part ces organes élastiques amortisseurs peuvent être rapidement changés pour modifier la raideur du dispositif.

Le capteur de force CEij placé sur chaque organe de liaison OLij permet de calculer la force axiale dans chaque organe de liaison et par conséquent de mesurer les forces exercées sur le plateau supérieur lors de l'assemblage effectué par le robot porteur. L'ordinateur 30 dispose alors d'algorithmes qui permettent de calculer les déplacements du support supérieur qui vont permettre la réalisation de l'assemblage. On effectue donc une commande des moteurs MOTij qui sont, dans cet exemple, des moteurs-couples par retour d'effort. Dans cette commande, le vecteur des forces s'exerçant au niveau de chaque emplacement d'articulation est converti dans un trièdre orthonormé en une force cartésienne qui est transmis à l'ordinateur 30 qui calcule à l'aide de l'algorithme de retour d'effort les corrections cartésiennes X à apporter à la position du plateau supérieur. Un transformateur de coordonnées, présent dans l'ordinateur 30, calcule alors les longueurs correspondantes des organes de liaison et transmet cette consigne au contrôleur 31 qui commande les moteurs MOTij.

Chaque moyen de sécurité MSij permet l'arrêt des moteurs lorsque la vis sans fin correspondante TFij atteint sa course minimum ou maximum. Ce moyen de sécurité coupe l'alimentation du moteur correspondant et assure un freinage en douceur de la vis sans fin TFij. Pour ce faire, lorsque l'organe de coulissement OCij s'approche de sa,fin de course maximum la présence de la lame OCij est détectée par l'interrupteur magnétique IMij fixé sur le support auxiliaire qui coupe le courant moteur. De même, lorsque l'organe de coulissement avance vers le plateau de base PB de façon à ramener l'articulation inférieure Alij vers le plateau inférieur PI, la présence de la lame CO'ij est détectée par le second interrupteur magnétique IM'ij qui coupe également le courant moteur. La déformation de ces lames élastiques sur le support auxiliaire SAij assure alors le freinage de l'organe de coulissement OCij et de la vis sans fin TFij. De plus, un interrupteur supplémentaire, au niveau du contrôleur 31, permet de court-circuiter les sécurités pour ramener manuellement le plateau supérieur dans la zone permise.

L'invention peut comporter des variantes, notamment les suivantes :
- les directions de coulissement peuvent être quelconques, comme par exemple parallèles au plateau inférieur et concourantes en son centre, et notamment elles peuvent être différentes d'un emplacement de liaison à un autre;
- suivant la direction de coulissement utilisée et/ou les types de moyens moteurs utilisés, l'homme de l'art pourra utiliser différents types d'articulations inférieures et différents types d'agencements pour les emplacements d'articulation en accord avec la cinématique du dispositif.

Bien entendu, certains des moyens décrits ci-dessus peuvent être omis dans les variantes où ils ne servent pas.

## Revendications

1. Dispositif articulé comprenant un plateau inférieur (PI), un plateau supérieur (PS) et un ensemble de commande de la position relative des deux plateaux (PI, PS), ledit ensemble de commande comportant :
- trois paires (EAi) d'emplacements d'articulation rapprochés (EAij) situées sur le plateau supérieur (PS),
- trois paires (PLi) d'emplacements de liaison rapprochés (PLij) situées sur le plateau inférieur (PI),
- six organes de liaison (OLij) possédant chacun une extrémité supérieure articulée sur l'un respectif des emplacements d'articulation (EAij), et une extrémité inférieure reliée par une articulation inférieure (Alij) à l'un respectif des emplacements de liaison (PLij), de telle sorte que deux emplacements d'articulation (EAij$_1$, EAij$_2$) d'une même paire (EAi) sont reliés par deux organes de liaison (OLi$_1$j$_2$, OLi$_2$j$_1$), aux deux emplacements de liaison consécutifs (PLi$_1$j$_2$, PLi$_2$j$_1$), qui sont respectivement les plus proches desdits deux emplacements d'articulation (EAij$_1$, EAij$_2$), et qui appartiennent à deux paires d'emplacements de liaison différentes (PLi$_1$, PLi$_2$),
- des moyens moteurs (MOTij, TFij) pour faire varier indépendamment sur commande la position d'un emplacement d'articulation (EAij) par rapport au plateau inférieur (PI),
caractérisé en ce que chaque organe de liaison (OLij) a une longueur sensiblement constante et en ce que l'ensemble de commande comprend, en outre, six organes de coulissement (OCij) respectivement reliés, d'une part, aux six emplacements de liaison (PLij) et, d'autre part, par l'intermédiaire des six articulations inférieures (Alij), aux extrémités inférieures des six organes de liaison (OLij), chaque organe de coulissement (OCij) étant propre à coulisser relativement au plateau inférieur (PI) selon une direction de coulissement prédéterminée (DC) sous l'action des moyens moteurs (MOTij, TFij).

2. Dispositif selon la revendication 1, caractérisé en ce que toutes les directions de coulissement (DC) sont sensiblement parallèles.

3. Dispositif selon la revendication 2, caractérisé en ce que toutes les directions de coulissement (DC) sont, en outre, sensiblement orthogonales au plan contenant les trois paires d'emplacements de liaison (PLi) situées sur le plateau inférieur (PI).

4. Dispositif selon la revendication 3, caractérisé en ce que chaque organe de liaison (OLij) est susceptible, sous l'action des moyens moteurs (MOTij, TFij), d'être positionné dans un cône de révolution ayant pour axe ladite direction de coulissement (DC) et pour sommet ladite articulation inférieure (Alij).

5. Dispositif selon la revendication 4, caractérisé en ce que l'ensemble de commande comprend des moyens de blocage (EAi, Alij) pour bloquer chaque organe de coulissement (OCij) en rotation autour de la direction de coulissement (DC).

6. Dispositif selon la revendication 5, caractérisé en ce que les moyens de blocage comprennent des moyens de blocage supérieurs (ARi, AR'i, AR"i, 62j, 61j) pour bloquer, au niveau de l'emplacement d'articulation correspondant (EAi), chaque organe de liaison (OLi₁j₂, OLi₂j₁) en rotation autour d'un axe reliant ses extrémités inférieure et supérieure et en ce que chaque articulation inférieure (Alij) est un joint de cardan constituant des moyens de blocage inférieurs.

7. Dispositif selon l'une des revendications 5 et 6, caractérisé en ce que chaque organe de coulissement (OCij) possède des moyens filetés (ECij), en ce que les moyens moteurs comprennent six vis sans fin (TFij) associées respectivement aux six organes de coulissement (OCij) et respectivement mues en rotation autour de leur axe longitudinal par six moteurs (MOTij), et en ce que les moyens filetés (ECij) d'un organe de coulissement et la vis sans fin associée (TFij) coopèrent pour permettre le coulissement de l'organe de coulissement.

8. Dispositif selon la revendication 7, caractérisé en-ce que chaque organe de coulissement (OCij) comprend un tube cylindrique creux (24j), possédant une première extrémité reliée à l'articulation inférieure (Alij) correspondante, ouvert à son autre extrémité, et en ce que les moyens filetés comprennent un écrou (ECij) fileté, solidairement lié en translation et en rotation à ladite autre extrémité et coopérant avec la vis sans fin (TFij).

9. Dispositif selon l'une des revendications 4 à 8, caractérisé en ce que chaque emplacement d'articulation (EAi) est propre à définir une première articulation supérieure (69i) possédant un premier axe de rotation (ARi) sensiblement perpendiculaire au plan contenant les trois paires d'emplacements d'articulation (EAi), et une deuxième articulation supérieure (80i) possédant un deuxième axe de rotation (AR'i) sensiblement orthogonal audit premier axe de rotation (ARi) et concourant avec ledit premier axe de rotation (ARi) et un troisième axe de rotation (AR"i) sensiblement orthogonal aux deux premiers axes et concourant avec ceux-ci ce qui permet d'obtenir trois degrés de liberté en rotation.

10. Dispositif selon l'une des revendications précédentes, caractérisé en ce que les trois points milieu (MIAi) des trois paires d'emplacements d'articulation (EAi) sont situés respectivement sensiblement aux trois sommets d'un premier triangle (EQI).

11. Dispositif selon l'une des revendications précédentes, caractérisé en ce que les six emplacements de liaison (PLi) sont situés sur un cercle (C).

12. Dispositif selon les revendications 10 et 11, caractérisé en ce que le premier triangle (EQ1) est équilatéral, et en ce que dans une position de repos, les trois points (MILi) situés respectivement au milieu des trois arcs de cercle reliant les deux emplacements de liaison respectifs des trois paires (PLi), sont placés respectivement sensiblement aux trois sommets d'un second triangle équilatéral (EQ2), décalé par rapport au premier triangle équilatéral (EQ1) d'un angle d'environ 60° autour de l'axe reliant les isobarycentres respectifs des deux triangles équilatéraux (EQ1, EQ2).

13. Dispositif selon l'une des revendications précédentes, caractérisé en ce que les deux emplacements d'articulation (EAij) de chaque paire (EAi) sont confondus.

14. Dispositif selon l'une des revendications précédentes, caractérisé en ce que l'ensemble de commande comprend des moyens de détection (PCij, CEij) d'au moins une grandeur mécanique représentative de l'état du dispositif.

15. Dispositif selon la revendication 14, caractérisé en ce que l'ensemble de commande comprend des moyens de traitement (MT) recevant les informations des moyens de détection (PCij, CEij) et commandant les moyens moteurs (MOTij, TFij).

16. Dispositif selon l'une des revendications précédentes, caractérisé en ce que l'ensemble de commande comprend six organes élastiques amortisseurs (OEAij) reliés respectivement aux six organes de liaison (OLij).

17. Dispositif selon l'une des revendications précédentes, caractérisé en ce qu'une partie des moyens moteurs (MOTij) est fixée sur un plateau de base (PB) solidaire du plateau inférieur (PI) le plateau inférieur (PI) étant situé entre le plateau de base (PB) et le plateau supérieur (PS).

**Patentansprüche**

1. Gelenkvorrichtung, welche eine untere Platte (PI), eine obere Platte (PS) und eine Antriebseinheit für die jeweilige Stellung der beiden Platten (PI, PS) enthält, wobei diese Einheit folgende Elemente aufweist:
- drei Paare (EAi) einander benachbarter Gelenkstellen (EAij), die auf der oberen Platte (PS) liegen,
- drei Paare (PLi) einander benachbarter Verbindungsstellen (PLij), die auf der unteren Platte (PI) liegen,
- sechs Verbindungselemente (OLij), von denen jedes ein oberes, gelenkiges Ende auf einer der Gelenkstellen (EAij) und ein unteres Ende besitzt, das durch ein unteres Gelenk (ALij) mit einer der jeweiligen Verbindungsstellen (PLij) verbunden ist, so daß zwei Gelenkstellen (EAij1, EAij2) eines Paares (EAi) durch zwei Verbindungselemente (OLi1j2, OLi2j1) mit den beiden folgenden Verbindungsstellen (PLi1j2, PLi2j1) verbunden sind, die jeweils den Gelenkstellen (EAij1, EAij2) am nächsten gelegen sind, und die zu zwei verschiedenen Verbindungsstellenpaaren (PLi1, PLi2) gehören, und
- Antriebselemente (MOTij, TFij), die dazu dienen, auf Befehl die Position einer Gelenkstelle (EAij) gegenüber der unteren Platte (PI) zu verändern,
dadurch gekennzeichnet, daß jedes Verbindungselement (OLij) eine konstante Länge aufweist, und die Antriebseinheit außerdem sechs Gleitelemente (OCij) umfaßt, die einerseits jeweils mit den sechs Verbindungsstellen (PLij) und anderseits durch die sechs unteren Gelenke (Alij) mit den unteren Enden der sechs Verbindungselemente (OLij) verbunden sind, wobei jedes Gleitelement (OCij) geeignet ist, bei Antrieb durch die Antriebselemente (MOTij, TFij) auf der unteren Platte (PI) entlang einer vorgegebenen Gleitrichtung (DC) zu gleiten.

2. Vorrichtung nach Anspruch 1, dadurch gekennzeichnet, daß alle Gleitrichtungen (DC) genau parallel sind.

3. Vorrichtung nach Anspruch 2, dadurch gekennzeichnet, daß alle Gleitrichtungen (DC) ferner orthogonal zu jener Ebene verlaufen, auf welcher die drei Verbindungsstellenpaare (PLi) liegen, die sich auf der unteren Platte (PI) befinden.

4. Vorrichtung nach Anspruch 3, dadurch gekennzeichnet, daß jedes Verbindungselement (OLij) geeignet ist, von den Antriebselementen (MOTij, TFij) angetrieben und in einem Drehkegel angeordnet zu werden, dessen Achse die obengenannte Gleitrichtung (DC) und dessen Scheitel das genannte untere Gelenk (Alij) ist.

5. Vorrichtung nach Anspruch 4, dadurch gekennzeichnet, daß die Antriebseinheit Blockiervorrichtungen (EAi, Alij) zum Blockieren der Gleitelemente (OCij) enthält, welche sich um die Gleitachse (DC) drehen.

6. Vorrichtung nach Anspruch 5, dadurch gekennzeichnet, daß die Blockiervorrichtungen obere Blockierelemente (ARi, AR'i, AR''i, 62j, 61j) enthalten, um an der entsprechenden Gelenkstelle (EAi) jedes Verbindungselement (OLi1j2, OLi2j1) zu blockieren, welches sich um eine Achse dreht, die dessen oberes und unteres Ende verbindet, und daß jedes untere Gelenk (Alij) ein Wellengelenk ist, welches die unteren Blockierelemente bildet.

7. Vorrichtung nach einem der Ansprüche 5 und 6, dadurch gekennzeichnet, daß jedes Gleitelement (OCij) Gewindevorrichtungen (ECij) besitzt, die Antriebselemente sechs Schnecken (TFij) enthalten, welche jeweils mit den sechs Gleitelementen (OCij) verbunden sind, und jeweils von sechs Motoren (MOTij) um eine Längsachse gedreht werden, und die Gewindevorrichtungen (ECij) eines Gleitelementes und die zugehörige Schnecke (TFij) zusammenwirken, um die Gleitbewegung des Gleitelementes zu ermöglichen.

8. Vorrichtung nach Anspruch 7, dadurch gekennzeichnet, daß jedes Gleitelement (OCij) ein hohles, zylindrisches Rohr (24j) enthält, das an einem Ende mit dem entsprechenden unteren Gelenk (Alij) verbunden ist und das an seinem anderen Ende offen ist, und daß die Gewindeelemente eine Mutter (ECij) mit Gewinde besitzen, die verschiebbar und drehbar mit diesem anderen Ende verbunden ist und mit der Schnecke (TFij) zusammenarbeitet.

9. Vorrichtung nach einem der Ansprüche 4 bis 8, dadurch gekennzeichnet, daß jede Gelenkstelle (EAi) geeignet ist, ein erstes oberes Gelenk (69i) zu definieren, welches eine erste Drehachse (ARi) besitzt, die genau vertikal zu der Ebene steht, auf welcher sich die drei Gelenkstellenpaare (EAi) befinden, und ein zweites oberes Gelenk (80i) zu definieren, welches eine zweite Drehachse (AR'i) besitzt, die genau orthogonal zu dieser ersten Drehachse (ARi) steht und mit dieser ersten Drehachse (ARi) genau gleich verläuft, und eine dritte Drehachse (AR''i) zu definieren, die genau orthogonal zu diesen beiden ersten Achsen steht und mit ihnen gleich verläuft, wodurch bei der Rotation ein Spiel von drei Grad erzielt wird.

10. Vorrichtung nach einem der vorhergehenden Ansprüche, dadurch gekennzeichnet, daß die drei Mittelpunkte (MIAi) der drei Gelenkstellenpaare (EAi) jeweils genau auf den drei Spitzen eines ersten Dreiecks (EQ1) zu liegen kommen.

11. Vorrichtung nach einem der vorhergehenden Ansprüche, dadurch gekennzeichnet, daß die sechs Verbindungsstellen (PLi) auf einem Kreis (C) liegen.

12. Vorrichtung nach Anspruch 10 und 11, dadurch gekennzeichnet, daß das erste Dreieck (EQ1) ein gleichseitiges Dreieck ist und sich in Ruhestellung befindet, wobei die drei Punkte (MILi), die jeweils in der Mitte der drei Kreisbögen liegen, welche die beiden zugehörigen Verbindungsstellen der drei Paare (PLi) verbinden, jeweils genau auf den drei Spitzen eines zweiten gleichseitigen Dreieckes (EQ2) liegen, wobei dieses zweite Dreieck gegenüber dem ersten gleichseitigen Dreieck (EQ1) um einen Winkel von ca. 60° um eine Achse versetzt ist, welche die jeweiligen isobarischen Mittelpunkte der beiden gleichseitigen Dreiecke (EQ1, EQ2) verbindet.

13. Vorrichtung nach einem der vorhergehenden Ansprüche, dadurch gekennzeichnet, daß die beiden Gelenkstellen (EAij) jedes Paares (EAi) deckungsgleich sind.

14. Vorrichtung nach einem der vorhergehenden Ansprüche, dadurch gekennzeichnet, daß die Antriebseinheit Erfassungsvorrichtungen (PCij, CEij) enthält, deren mechanische Mindestgröße dem Zustand des Gerätes entspricht.

15. Vorrichtung nach Anspruch 14, dadurch gekennzeichnet, daß die Antriebseinheit Verarbeitungselemente (MT) enthält, welche die Informationen der Erfassungsvorrichtungen (PCij, CEij) aufnehmen und die Antriebselemente (MOTij, TFij) steuern.

16. Vorrichtung nach einem der vorhergehenden Ansprüche, dadurch gekennzeichnet, daß die Steuereinheit sechs elastische Dämpfungselemente (OEAij) beinhaltet, welche jeweils mit den sechs Verbindungselementen (OLij) verbunden sind.

17. Vorrichtung nach einem der vorhergehendenAnsprüche, dadurch gekennzeichnet, daß ein Teil der Antriebselemente (MOTij) auf einer Grundplatte (PB) befestigt ist, die drehfest mit der unteren Platte (PI) verbunden ist, welche sich zwischen der Grundplatte (PB) und der oberen Platte (PS) befindet.

## Claims

1. Articulated device comprising a lower plate (PI), an upper plate (PS), and an assembly for controlling the relative position of the two plates (PI, PS), the said control assembly comprising:
    – three pairs (EAi) of close-together articulation locations (EAij) situated on the upper plate (PS),
    – three pairs (PLi) of close-together linking locations (PLij) situated on the lower plate (PI),
    – six linking members (OLij), each having an upper end articulated on respectively one of the articulation locations (EAij), and a lower end connected by a lower articulation (Alij) to respectively one of the linking locations (PLij) in such a way that two articulation locations (EAij$_1$, EAij$_2$) of the same pair (EAi) are connected by two linking members (OLi$_1$j$_2$, OLi$_2$j$_1$) to the two consecutive linking locations (PLi$_1$j$_2$, PLi$_2$j$_1$) which are respectively closest to the said two articulation locations (EAij$_1$, EAij$_2$), and which belong to two pairs of different linking locations (PLi$_1$, PLi$_2$),
    – drive means (MOTij, TFij) for varying independently, on command, the position of an articulation location (EAij) with respect to the lower plate (PI),
characterised in that each linking member (OLij) has a substantially constant length, and in that the control assembly furthermore comprises six sliding members (OCij) which are respectively connected, on the one hand, to the six linking locations (PLij) and, on the other hand, via six lower articulations (Alij), to the lower ends of the six linking members (OLij), each sliding member (OCij) being capable of sliding relative to the lower plate (PI) in a predetermined direction of sliding (DC) under the action of the drive means (MOTij, TFij).

2. Device according to Claim 1, characterised in that all the directions of sliding (DC) are substantially parallel.

3. Device according to Claim 2, characterised in that all the directions of sliding (DC) are furthermore substantially orthogonal to the plane containing the three pairs of linking locations (PLi) situated on the lower plate (PI).

4. Device according to Claim 3, characterised in that each linking member (OLij) is capable, under the action of the drive means (MOTij, TFij), of being positioned in a cone of revolution having as its axis the said direction of sliding (DC) and as its apex the said lower articulation (Alij).

5. Device according to Claim 4, characterised in that the control assembly comprises locking means (EAi, Alij) for locking each sliding member (OCij) in rotation about the direction of sliding (DC).

6. Device according to Claim 5, characterised in that the locking means comprise upper locking means (ARi, AR'i, AR''i, 62j, 61j) for locking, at the level of the corresponding articulation location (EAi), each linking member (OLi$_1$j$_2$, OLi$_2$j$_1$) in rotation about an axis joining its lower and upper ends, and in that each lower articulation (Alij) is a universal joint constituting lower locking means.

7. Device according to either of Claims 5 and 6, characterised in that each sliding member (OCij) has threaded means (ECij), in that the drive means comprise six endless screws (TFij) associated respectively with the six sliding members (OCij) and respectively moved in rotation about their longitudinal axis by six motors (MOTij), and in that the threaded means (ECij) of a sliding member and the associated endless screw (TFij) interact in order to permit the sliding of the sliding member.

8. Device according to Claim 7, characterised in that each sliding member (OCij) comprises a hollow cylindrical tube (24j) having a first end connected to the corresponding low articulation (Alij) and open at its other end, and in that the threaded means comprise a threaded nut (ECij) linked integrally in terms of translational and rotational motion to the said other end and interacting with the endless screw (TFij).

9. Device according to one of Claims 4 to 8, characterised in that each articulation location (EAi) is capable of defining a first upper articulation (69i) having a first axis of rotation (ARi) substantially perpendicular to the plane containing the three pairs of articulation locations (EAi), and a second upper articulation (80i) having a second axis of rotation (AR'i) substantially orthogonal to the said first axis of rotation (ARi) and coinciding with the said first axis of rotation (ARi), and a third axis of rotation (AR''i) substantially orthogonal to the first two axes and coinciding with them, which makes it possible to obtain three degrees of freedom in rotation.

10. Device according to one of the preceding claims, characterised in that the three middle points (MIAi) of the three pairs of articulation locations (EAi) are situated respectively substantially at the three apices of a first triangle (EQ1).

11. Device according to one of the preceding claims, characterised in that the six linking locations (PLi) are situated on a circle (C).

12. Device according to Claims 10 and 11, characterised in that the first triangle (EQ1) is equilateral, and in that, in a rest position, the three points (MILi) situated respectively at the centre of the three arcs of a circle connecting the two respective linking locations of the three pairs (PLi) are placed respectively substantially at the three apices of a second equilateral triangle (EQ2) offset with respect to the first equilateral triangle (EQ1) by an angle of approximately 60° about the axis connecting the respective isobaric centres of the two equilateral triangles (EQ1, EQ2).

13. Device according to one of the preceding claims, characterised in that the two articulation locations (EAij) of each pair (EAi) are at one and the same point.

14. Device according to one of the preceding claims, characterised in that the control assembly comprises detection means (PCij, CEij) for at least one mechanical quantity representative of the condition of the device.

15. Device according to Claim 14, characterised in that the control assembly comprises processing means (MT) receiving the data from the detection means (PCij, CEij) and controlling the drive means (MOTij, TFij).

16. Device according to one of the preceding claims, characterised in that the control assembly comprises six resilient damping members (OEAij) connected respectively to the six linking members (OLij).

17. Device according to one of the preceding claims, characterised in that a part of the drive means (MOTij) is fixed to a baseplate (PB) integrally joined to the lower plate (PI), the lower plate (PI) being situated between the baseplate (PB) and the upper plate (PS).

EP 0 362 342 B1

FIG.1

FIG.3

13

PS

EA2

EA3                    EA1

OL30                                    OL21
OL31                                        OL20
AI30          OL10      OL11           AI21
AI31                                          AI20
                                              OC20
                    AI10        AI11
                              PL21        PL20
                                              PI
              PL10    OC11
                      PL11

1

PC10        PC11        TF20

            TF11

PCB

MOT20

MOT11

PB

# FIG.2

CEij

OLij

AIij

21j

22j

26j

25j

OEAij

20j

OCij

24j  PLij

V

27j

DBij

PI

PCij

TFij

0

**FIG.4**

20j 24j OCij PLij PI

PCij DBij ECij TFij

# FIG.5

# FIG.6

$$\underline{F\,I\,G.7}$$

$$\underline{F\,I\,G.8}$$